# EUROPEAN PATENT APPLICATION

(11) **EP 1 321 036 A2**
(43) Date of publication of application: **25.06.2003**
(21) Application number: 02258843.8
(22) Date of filing: 20.12.2002
(51) Int. Cl.: A01M 1/20, A61L 9/03

(54) **Illumination and air-treatment device**

(30) Priority: 22.12.2001 GB 0130818
(71) Applicant: Robert McBride Ltd, Middleton, Manchester M24 4DP (GB)
(72) Inventor: Smith, Nigel Peter, Devon, TQ12 6HH (GB)
(74) Representative: Newstead, Michael John

(57) **Abstract**

The invention provides a unit (5) which not only acts as a non-electric source of light, but also operates to repel and/or kill insects and the like.

In the form shown, the unit (5) comprises a holder (6) for a candle, in the form of a dish. A lid (8), which engages the upper rim of the dish, carries a pad (9) impregnated with an insecticide and/or repellant. The candle flame not only provides a source of light but also heats the lid (8) causing the insecticide to be volatilized off from the pad (9).

## Description

### Field of the Invention

This invention relates to an illumination and air treatment device and has been devised in particular, though not necessarily solely, as a device for use as a source of insecticide and/or repellant in locations where the use of an equivalent electric powered device is not possible or practicable.

### Background to the Invention

Consumers typically use candles or oil powered lanterns to provide a source of light for ambience, or in locations in which electrical power is not readily available. These are effective as light sources to a varying extent but are generally highly effective at providing a beacon for insects and other night life; which can often prove to be a source of discomfort. Candles are available which include insect repellant substances in the base candle material. However these types of candle have limited effectiveness and often the repellent is burnt in the candle flame before it can take effect. Accordingly the consumer must use other means to repel insects such as discrete insect repellants, insecticides and/or insect nets.

It is an object of this invention to provide a form of illumination and air-treatment device which addresses the problem set forth above; or which will at least provide a useful choice.

### Summary of the Invention

Accordingly, in a first aspect, the invention provides a device for use in providing illumination and air treatment, said device including:
a holder for non-electric source of light and heat;
a carrier for a source of air-treatment agent;
said carrier being located in relation to said holder so as to, in use, receive heat from a source of light and heat located in said holder.

Preferably said holder is constructed and arranged as part of, or to hold, a candle. Alternatively said holder is constructed and arranged as part of, or to hold, a liquid fuel powered lantern.

Preferably said carrier includes a surface part conductive to heat. Preferably said surface part is sized and configured to locate a mat or other porous member impregnated with an air-treatment agent.

Preferably said carrier is configured to, in use, support the operation of said source of light and heat.

In a second aspect the invention provides an illumination and air-treatment unit including
a device as set forth above;
a source of heat and light located in said holder; and
a source of air-treatment agent located on said carrier.

Many variations in the way the invention may be performed will present themselves to those skilled in the art upon reading the following description. The description which follows should not be regarded as limiting but rather, as an illustration only of one mode of performing the invention. Where possible, a description of any element or component should be taken as including any or all equivalents thereof whether or not specifically mentioned. The scope of the invention should be determined solely by the appended claims.

### Brief Description of the Drawings

One form of dispenser embodying the various aspects of the invention will now be described with reference to the accompanying drawing in which:
- Figure 1:: shows an isometric view of an illumination and air-treatment unit according to the invention; and
- Figure 2:: shows an enlarged view of a carrier included in the unit shown in Figure 1.

### Detailed Description of Working Embodiment

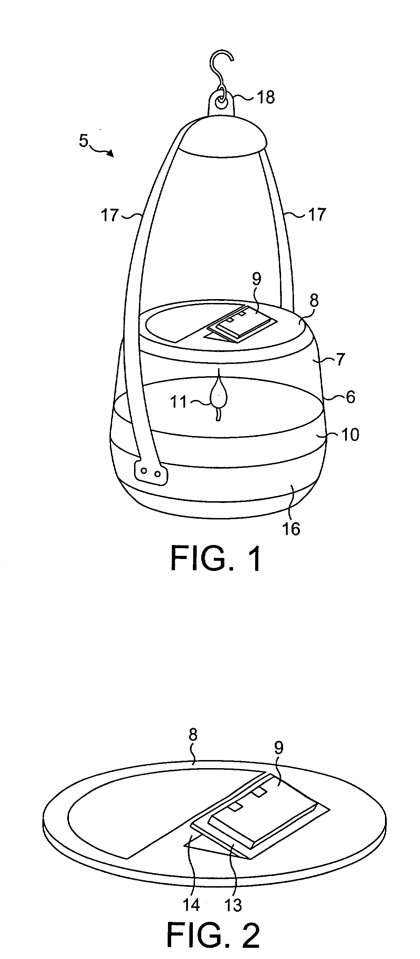

Referring to Figure 1, the invention provides an illumination and air treatment unit 5 which requires no form of external energy, such as electric power, to be effective.

In the form shown, the unit 5 comprises a holder 6 constructed and arranged to locate and retain a non-electric source 7 of light and heat. The unit 5 further includes a carrier 8 to mount a source 9 of air-treatment agent. The carrier 8 is located in relation to the holder so that the source 9 of air-treatment agent receives heat from the source 7 of heat and light, this heat then causing the air-treatment agent to be volatilized off into the surrounding atmosphere.

The source 7 of heat and light may, as shown, be a candle. Alternatively, the source 7 may be a liquid fuel powered lantern.

In the particular form shown, the holder 6 comprises a glass or other substantially transparent dish in which active candle material 10 is retained.
In the conventional manner, the candle also includes a wick 11 embedded in the material 10.

The active candle material 8 may be of any form able to generate light as well as sufficient heat to release the air-treatment agent. By way of example, the candle material 10 may be of the gel co-polymer type, the co-polymer being mixed with a mineral oil. Suitable co-polymers include those sold under the trade mark KRATON, grade 1650. Suitable mineral oils include white mineral oil. The composition may comprise about 8% by weight of co-polymer, about 88% mineral oil and the balance fragrance.

The carrier 8, as shown, takes the form of a metal lid which rests on the upper rim of the dish 6. The lid is preferably formed from aluminium or like heat conductive metal and has a surface part 13 on which the source 9 of air-treatment agent is mounted. In the embodiment herein described the surface part 13 is angled up out of the general plane of the metal lid, thus defining air gap 14. The air-gap 14 supports the operation of the candle by enabling the ingress of air into the dish 6 to support combustion.

Using a lid formed from aluminium, a candle composition as described above, and with the lid positioned 30mm from the surface of the gel when the candle is fresh, temperatures in the order of 120 to 140°C can be achieved on the carrier 8, after 30 minutes, using an air gap 13 of about 10mm. Even when the candle material 10 is half depleted, temperatures in excess of 100°C can still be achieved on the lid.

Given the temperatures available at the carrier 8, a suitable source 9 of air-treatment agent comprises a cellulose pad or mat impregnated with bio-allethrin, a common form of pyrethroid. The bio-allethrin volatilizes off from the cellulose pad or mat, when the pad or mat is subjected to heating. These mats are well known and are commonly available for use in electric powered evaporators, as mosquito insecticides and repellants.

The combination of dish 6 and lid 8, each with its own active material, may comprise an operating unit which sits on a substantially horizontal support surface. Alternatively, and as shown, the dish 6, with attached lid 8, may be located in a suspension bracket 15 defined by a ring 16 attached to the lower ends of upstanding arms 17. The upper ends of arms 17 may meet at suspension point 18, the suspension point 18 allowing the unit to be suspended in the manner of a conventional lantern.

It will thus be appreciated that the present invention, at least in the case of the working embodiment described above, provides a simple yet effective form of illumination and air-treatment device which can be used in the absence of electric power, provides a source of light, yet also has the ability to effectively kill or repel insects and other night life.

## Claims

1. A device for use in providing illumination and air treatment, said device including:
a holder (6) for non-electric source of light and heat;
a carrier (8) for a source of air-treatment agent;
said carrier (8) being located in relation to said holder (6) so as to, in use, receive heat from a source of light and heat (7) located in said holder.

2. A device as claimed in claim 1 wherein said holder (6) is constructed and arranged as part of, or to hold, a candle.

3. A device as claimed in claim 1 wherein said holder is constructed and arranged as part of, or to hold, a liquid fuel powered lantern.

4. A device as claimed in any one of claims 1 to 3 wherein said carrier (8) includes a surface part (13) conductive to heat.

5. A device as claimed in claim 4 wherein said surface part (13) is sized and configured to locate a mat or other porous member (9) impregnated with an air-treatment agent.

6. A device as claimed in any one of the preceding claims wherein said carrier is configured to, in use, support the operation of said source of light and heat.

7. An illumination and air-treatment unit including
a device as set forth above;
a source of heat and light (7) located in said holder (6); and
a source (9) of air-treatment agent located on said carrier.

8. An illumination and air-treatment device when constructed arranged and operable substantially as herein described with reference to, and as illustrated in, the accompanying drawings.
